Europäisches Patentamt

(19) European Patent Office

Office européen des brevets

(11) Publication number: **0 186 368 B1**

## EUROPEAN PATENT SPECIFICATION

(45) Date of publication of patent specification: **03.04.91**   (51) Int. Cl.⁵: **A61K 39/215**

(21) Application number: **85308952.2**

(22) Date of filing: **10.12.85**

The file contains technical information submitted after the application was filed and not included in this specification

(54) Biological preparations.

(30) Priority: **11.12.84 GB 8431253**

(43) Date of publication of application:
**02.07.86 Bulletin 86/27**

(45) Publication of the grant of the patent:
**03.04.91 Bulletin 91/14**

(84) Designated Contracting States:
**AT BE CH DE FR GB IT LI LU NL SE**

(56) References cited:
**EP-A- 0 030 063**

**BIOLOGICAL ABSTRACTS, vol. 75, no.12, 1983, p. 9221, ref.no. 88105; Philadelphia, US. W.W. MARQUADT et al.: "Comparison of cilliary activity and virus recovery from tracheas of chickens and humoral immunity after inoculation with aerotypes of avian infectious bronchitis virus"**

**BIOLOGICAL ABSTRACTS, vol. 76, no. 7, 1983, p. 5372, ref.no. 49356;Philadelphia, US. L.M.TIMMS et al.: "Cell mediated and humoral imumune response of chickens to inactivated oil-emulsion infectious bronchitis vaccine".**

(73) Proprietor: **PITMAN-MOORE, INC.**
**2315 Sanders Road**
**Northbrook Illinois 60062(US)**

(72) Inventor: **Box, Phillip George Mount View Pyrton**
**Watlington**
**Oxfordshire OX9 5AP(GB)**
Inventor: **Holmes, Harvey Charles**
**35 Sefton Avenue Mill Hill**
**London NW7 3QE(GB)**

(74) Representative: **Holmes, Michael John et al**
**Frank B. Dehn & Co. European Patent Attorneys Imperial House 15-19 Kingsway**
**London, WC2B 6UZ,(GB)**

BIOLOGICAL ABSTRACTS, vol. 67, no. 3,
1979, p. 1611, ref. no. 16459; Philadelphia, US.
H.C.HOLMES: " The resistance of reinfection
of tracheal organ cultures from chickens
previously infected with avian infectious
bronchitis virus" .

BIOLOGICAL ABSTRACTS, vol. 72, no. 4,
1981, p. 2556, ref. no. 24308; Philadelphia, US.
P.J. WYETH et al.: "Immune responses of
breeding chickens to trivalent oil emulsion
vaccines: Responses to Newcastle disease
and infectious bursa disease" .

Dtsch. tierärztl. Wschr., Vol.93, 1986,
pp.481-486 .

## Description

This invention relates to novel vaccines for immunising poultry against infectious bronchitis.

Infectious bronchitis is a highly contagious respiratory disease of chickens which, in the case of laying hens, may also adversely affect egg production and quality. The use of infectious bronchitis vaccines for immunisation of poultry has now been established for many years.

In general, the vaccines contain an infectious bronchitis virus of a particular serotype such as Connecticut or Massachusetts serotypes, the latter being more common in Great Britain and Europe. Combination of different infectious bronchitis serotypes has also been tried, but protection against the respective serotypes is not always optimal.

More recently, inactivated infectious bronchitis vaccines containing variant, i.e. new serologically distinct, strains of infectious bronchitis virus have been introduced but they require poultry to be initially immunised or "primed" with a live vaccine; hitherto it has been believed that such "priming" had to be effected using a live vaccine comprising a virus of the same strain as the inactivated virus.

We have now found that inactivated vaccines derived from a novel variant strain of avian infectious bronchitis virus and strains which cross-react with this particular variant strain offer protection against such serologically related variant strains and furthermore, immunisation with such vaccines surprisingly does not require initial vaccination or "priming" of the poultry with a live vaccine of the same strain. This enables "priming" to be effected using heterologous live vaccines which may be well tried, conventional live vaccines of established safety and effectiveness.

The novel avian infectious bronchitis variant virus strain was isolated from broiler chickens and has been deposited at the Central Veterinary Laboratory, New Haw at Weybridge, United Kingdom registered under the number VLO 10110/AVI/9 and also at the Institut Pasteur, Paris, France registered under the number I-400 deposited on 11th December 1984.

This strain, hereinafter referred to as VLO 10110/AVI/9, was shown in virus neutralisation tests to be related but not identical to the Dutch D207 strain but is distinct from the conventional viruses of the Massachusetts serotype commonly used in infectious bronchitis vaccines. By means of cross-neutralisation tests carried out in tracheal organ cultures, it was found that the isolate is not neutralised by antiserum to infectious bronchitis virus M41 or to the H120 vaccine strains of infectious bronchitis virus both of which are of the Massachusetts serotype. Table 1 summarises the results of the various virus neutralisation (VN) tests using the constant-virus variable-serum method. The results indicate that the new variant virus VLO 10110/AVI/9 is distinct serologically and does not belong to the Massachusetts serotype. The oil emulsion vaccines referred to in Table 1 contain the Massachusetts M41 strain and/or the VLO 10110/AVI/9 variant strain of avian infectious bronchitis virus.

## TABLE 1

### VN TESTS ON INFECTIOUS BRONCHITIS VIRUS SERA

| Group | Live "Primer" | Oil Emulsion Vaccine | Reciprocal titres* | |
|-------|---------------|----------------------|---------------------|------|
| | | | VLO 10110/AVI/9 variant strain | M41 |
| A | VLO 10110/AVI/9 variant strain | – | 180 | <10 |
| B | – | M41 | <10 | 160 |
| C | – | VLO 10110/AVI/9 variant strain | 25 | <10 |
| D | H120 | M41 | <10 | >640 |
| E | H120 | M41 + VLO 10110/AVI/9 variant strain (in equal parts) | 394 | >640 |

\* mean of two assays

According to one feature of the invention, therefore, we provide novel vaccines containing inactivated avian infectious bronchitis virus VLO 10110/AVI/9 and/or an inactivated virus strain which cross-reacts significantly therewith, the immune response to which in birds is enhanced by "priming" with a live heterologous strain of avian infectious bronchitis virus.

As indicated above, we have found that an inactivated vaccine based on the novel variant strain VLO 10110/AVI/9 of infectious bronchitis virus and/or a strain serologically related thereto does not require poultry to have been initially vaccinated or "primed" with a live vaccine of the same strain. Instead, and unexpectedly we have found that "priming" with a heterologous live vaccine based on a conventional strain (example H120 vaccine) is sufficient to ensure that subsequent vaccination with the vaccine based on the inactivated variant strain gives a boosted immune response. Thus we have found that "priming" with a live attenuated virus vaccine of the Massachusetts serotype such as a live vaccine to any conventional strain of the Massachusetts serotype H120, H52 or M41, or mild Massachusetts (MM) is sufficient to ensure that subsequent vaccination with the inactivated variant strain vaccine to VLO 10110/AVI/9 and/or serologically related strains gives a boosted immune response.

The inactivated vaccines to the variant strain VLO 10110/AVI/9 and/or strains cross-reacting significantly therewith may be prepared by conventional means, for example by growing the virus in embryonated eggs. The infectious allantoic fluid having reached a suitable titre e.g. approximately $10^{8.0}$ median ciliostatic doses/ml (8.0 $\log_{10}$ $CD_{50}$/ml wherein $CD_{50}$/ml is as defined in J.K.A. Cook et. al., 1976 Archives of Virology, 50 , 109-118) may then be harvested and inactivated using inactivating agents such as formaldehyde or $\beta$-propiolactone. After inactivation the virus may be concentrated and the pH may be adjusted if necessary. The inactivated antigen in an aqueous medium may be mixed with an adjuvant such as aluminium hydroxide or incorporated into an oil emulsion comprising an oil, e.g. paraffin oil, an oil-soluble surfactant, and a water soluble surfactant, the ratio of oil to aqueous phase generally being in the range 2-5:1.

Purified paraffin oils which may be used in an adjuvant emulsion include, for example, proprietary products such as Drakeol, Marcol or Puremore, though most grades of liquid paraffin are suitable. An oil such as squalane may also be employed.

The most preferred ratio of oil to aqueous phase is about 4:1, the resulting emulsion being stable and of a suitable viscosity for injection.

The oil-soluble surfactant is preferably a monoester of a sugar alcohol anhydride, for example sorbitan or mannitan, with a long-chain fatty acid such as oleic, stearic or lauric acid. Arlacel 80® or Span 80® - (sorbitan mono-oleate) and Arlacel A® (mannitan mono-oleate) are particularly suitable.

The water-soluble surfactant is preferably a polyoxyethylene derivative of a monoester of a sugar alcohol anhydride, such as sorbitan with a long-chain fatty acid such as lauric, oleic or stearic acid. The polyoxyethylene chain preferably contains about 20 oxyethylene units. Tween 80® (polyoxyethylene (20) sorbitan mono-oleate) is particularly suitable.

The concentration of the oil-soluble surfactant in the oil phase is preferably in the range 8-12% (w/v), advantageously about 10% (w/v). The concentration of the water-soluble surfactant in the water-phase is preferably in the range of 4 to 4.5% (w/v), advantageously about 4.25% (w/v).

The vaccine may advantageously contain inactivated virus obtained from $10^6$ to $10^8$ median ciliostatic doses (6 to 8 $\log_{10}$ $CD_{50}$) of the variant strain VLO 10110/AVI/9 (and/or a serologically related strain) per dose of vaccine.

The inactivated vaccines will usually be given to the birds at the point of lay at an age of approximately 16-20 weeks by subcutaneous or intramuscular injection, preferably by the intramuscular route. One dose of the inactivated vaccine is usually sufficient and may, for example, consist of 0.25 to 1.00 ml, e.g. 0.5 ml, of the above vaccine, such a dose advantageously containing the antigenic equivalent of $10^6$ to $10^8$ median cilostatic doses (6 to 8.0 $\log_{10}$ $CD_{50}$)of the variant virus strain.

The inactivated vaccines may contain, in addition to the inactivated variant strain VLO 10110/AVI/9 and/or a strain which cross-reacts significantly therewith one or more additional inactivated virus strains such as those of the Massachusetts serotype for example M41, other strains of infectious bronchitis virus or other viruses such as Newcastle disease virus, infectious bursal disease virus, adeno viruses such as EDS 76 virus, reoviruses, and other viruses. Thus, for example, such vaccines include a monovalent oil emulsion vaccine containing inactivated VLO 10110/AVI/9 alone, a bivalent oil emulsion vaccine containing both inactivated VLO 10110/AVI/9 and inactivated Massachusetts M41, a trivalent oil emulsion vaccine containing VLO 10110/AVI/9, inactivated Massachusetts M41 and Newcastle disease virus or a tetravalent oil emulsion vaccine containing VLO 10110/AVI/9, inactivated Massachusetts M41, Newcastle disease virus and infectious bursal disease virus.

As indicated above, the recipients of the inactivated vaccines of the invention may be "primed" using a conventional live infectious bronchitis virus vaccine, for example live attenuated virus vaccine containing an avian infectious bronchitis virus of the Massachusetts serotype such as a live vaccine to a conventional H120, H52, M41 or MM virus. This is sufficient to ensure that subsequent vaccination with an emulsion variant strain vaccine gives a boosted immune response.

Infectious bronchitis virus H120 vaccine is preferred as the "priming" vaccine,

The "priming" vaccine is given to chickens at up to 12 weeks of age, preferably 3 weeks of age. In addition an optional second "priming" dose or further doses may be given. The vaccine may contain at least 3 $\log_{10}$ median egg infectious doses ($EID_{50}$) of live attenuated avian infectious bronchitis virus per bird dose. The vaccine may be administered in the drinking water, by eye drop, intranasally or by aerosol spray. In general, the inactivated vaccine should be given at least 4 weeks, more preferably about 13 weeks, after the "priming" vaccine.

According to a still further feature of the invention, we provide a kit comprising separately said inactivated virus and said live virus vaccines. The kit comprises multiple dosage units of each of the respective vaccines. Preferably, 500 or 1000 dosage units of each vaccine may be present in the kit.

According to yet another feature of the invention, we provide the use of inactivated avian infectious bronchitis virus VLO 10110/AVI/9 and/or a strain cross-reacting significantly therewith, to prepare a vaccine for use in vaccinating birds against avian infectious bronchitis in combination with an initial vaccination with a live vaccine containing a live heterologous avian infectious bronchitis virus.

The following examples illustrate the invention.

Example 1

(a) Preparation of monovalent oil emulsion vaccine containing inactivated strain VLO 10110/AVI/9

Ten day old SPF embryonated chicken eggs were inoculated via the allantoic cavity with 3-4 $log_{10}$ $CD_{50}$ of infectious bronchitis virus strain VLO 10110/AVI/9. After 70 hours incubation at 37°C, the allantoic fluid was harvested. A preservative was added (0.013% thiomersal) and after clarification by low speed centrifugation, virus infectivity was inactivated by incubation with 0.05% $\beta$-propiolactone for 2 hours at 37°C. The inactivated allantoic fluid was concentrated by ultrafiltration such that it contained at least the equivalent of $10^8$ $CD_{50}$/ml. Polysorbate 80 was dissolved in the inactivated viral antigen preparation to a concentration of 4.25%. This was then mixed with a light mineral oil (USP) containing 10% sorbitan monooleate BP in the ratio of 1:4 and emulsified. The vaccine was stored at 4°C.

(b) Immunisation

One dose, containing at least 3 $log_{10}$ $EID_{50}$ of H120 live vaccine, was administered to chickens intranasally at 3 weeks of age. At 16 weeks of age 0.5 ml of the VLO 10110/AVI/9 oil emulsion vaccine (antigenic equivalent 7.0 $log_{10}$ $CD_{50}$) was administered to each bird intramuscularly. Mean haemagglutination inhibition (HI) antibody titres to M41 of 7.0 $log_2$ and to D274 of 8.3 $log_2$ were obtained 8 weeks after vaccination with the oil emulsion vaccine.

Example 2

Preparation of bivalent oil emulsion vaccine containing inactivated strain VLO 10110/AVI/9 and strain M41

(a) Two batches of ten day old SPF embryonated chicken eggs were inoculated via the allantoic cavity with 3-4 $log_{10}$ $CD_{50}$ of infectious bronchitis virus strain VLO 10110/AVI/9 or strain M41. After 70-72 hours incubation at 37°C, the allantoic fluid from each batch of eggs was harvested separately. A preservative was added to each (0.013% thiomersal) and after clarification by low speed centrifugation, virus infectivity was inactivated by incubation with 0.05% $\beta$-propiolactone for 2 hours at 37°C. The two batches of inactivated allantoic fluid were concentrated by ultrafiltration. Polysorbate 80 was dissolved in the inactivated viral antigen preparations to a concentration of 4.25%. These were then mixed with a light mineral oil (USP) containing 10% sorbitan monooleate BP in the ratio 1:4 and emulsified. The two emulsions were then mixed together in equal volumes and the bivalent vaccine stored at 4°C.

(b) Similarly, polyvalent vaccines may be prepared using the above technique but incorporating, in addition, inactivated Newcastle disease virus or inactivated Newcastle disease virus and infectious bursal disease virus.

(c) Immunisation

One dose, containing at least 3 $log_{10}$ $CD_{50}$ of H120 live vaccine was administered to chickens intranasally at 3 weeks of age. At 16 weeks of age 1ml of the bivalent oil emulsion vaccine of (a) above (antigenic equivalent 7.0 $log_{10}$ $CD_{50}$ of each virus strain) was administered to each bird intramuscularly. Mean haemagglutination inhibition (HI) antibody titres to M41 of >10.7 $log_2$ and D274 of 9.0 $log_2$ were obtained 8 weeks after vaccination with the oil emulsion vaccine.

Example 3

Comparative test in "primed" and "unprimed" birds

The monovalent oil emulsion vaccine prepared as in Example 1 above, or an equivalent M41 monovalent vaccine or a bivalent oil emulsion vaccine prepared as in Example 2(a) above containing both viral antigens was administered both to 16 week old chickens that had been "primed" with live H120 (a vaccine of the Massachusetts serotype widely used commercially) and to 16 week old chickens which had not been "primed". Haemagglutination inhibition (HI) antibody titres were measured at 4 and 8 weeks after vaccination. Boosted immune responses and relatively high antibody titres to the variant virus were found in birds vaccinated with the monovalent VLO 10110/AVI/9 or the bivalent oil emulsion vaccine which had previously been "primed" with the conventional H120 live vaccine. The antibody titres were considerably higher than the titres induced in "unprimed" birds. (See Table 2). It will be appreciated that the corresponding boosting of antibody titres in respect of the M41 virus when "primed" with H120 vaccine is conventional in that M41 and H120 are of the same serotype.

## TABLE 2

## RECIPROCAL $LOG_2$ MEAN HI ANTIBODY TITRES IN CHICKENS

| Live Virus | Oil Emulsion Vaccine | HI titres to M41 | | HI titres to D274* | |
|---|---|---|---|---|---|
| | | 20 weeks | 24 weeks | 20 weeks | 24 weeks |
| None | M41 | 7.3 | 9.2 | 4.6 | 4.8 |
| H120 | M41 | >10.6 | >10.2 | 5.1 | 7.1 |
| None | VLO 10110/AVI/9 | 3.9 | 5.3 | 5.9 | 6.6 |
| H120 | VLO 10110/AVI/9 | 6.6 | 7.0 | 7.2 | 8.3 |
| None | VLO 10110/AVI/9 + M41 | 7.3 | 7.8 | 6.3 | 6.7 |
| H120 | VLO 10110/AVI/9 + M41 | >10.6 | >10.7 | >8.1 | 9.0 |

\* D274 virus is a similar strain to D207 and was used for the HI test because it produces a high titred haemagglutinin.

Example 4

Hubbard Comet pullets were reared under commercial conditions on an isolated site. At 0, 4 and 8 weeks of age they were administered live H120 infectious bronchitis vaccine.

At 15 weeks of age, serum samples were examined and shown to be free of antibodies to 'variant' strains of infectious bronchitis virus.

The birds were transferred to individual bird laying cages in an isolation suite. Half the birds were vaccinated with the bivalent oil emulsion vaccine of Example 2 containing inactivated strain VLO 10110/AVI/9 + inactivated strain M41 and half received no further vaccine. Individual bird egg production including egg colour was recorded daily.

At 30 weeks of age, the birds were challenged with a fine aerosol of strain VLO 10110/AVI/9.

Vaccination of commercial pullets with a combination of live H120 and inactivated bivalent oil emulsion vaccine was successful in preventing the serious drop in egg production and adverse changes in shell quality that occurred in birds not so vaccinated when challenged at 30 weeks old.

## Claims

1. Vaccines containing inactivated avian infectious bronchitis virus strain deposited with the Institut Pasteur under the accession number I-400 and/or an inactivated virus strain which cross-reacts significantly with said strain the immune response to which in birds is enhanced by priming with a live heterologous strain of avian infectious bronchitis virus.

2. A vaccine as claimed in claim 1 wherein the inactivated avian infectious bronchitis virus is I-400.

3. A vaccine as claimed in claim 1 or claim 2 wherein the inactivated antigen in an aqueous medium is mixed with aluminium hydroxide or an oil emulsion comprising an oil, an oil-soluble surfactant and a water-soluble surfactant.

4. A vaccine as claimed in claim 3 which comprises as an adjuvant an oil emulsion wherein the oil is a paraffin oil or squalane.

5. A vaccine as claimed in claim 3 or claim 4 which comprises as an adjuvant an oil emulsion wherein the ratio of oil to aqueous phase is about 4:1.

6. A vaccine as claimed in any one of claims 1 to 5 which contains inactivated virus obtained from 6 to 8 $\log_{10}$ $CD_{50}$ of the variant strain I-400 and/or said cross-reacting strain per dose of vaccine.

7. A vaccine as claimed in any one of claims 1 to 6 which contains, in addition to inactivated avian infectious bronchitis virus strain I-400 and/or an inactivated virus strain which cross-reacts significantly with said strain, one or more additional inactivated virus strains.

8. A vaccine as claimed in any of claims 1-7 which contains in addition to said inactivated avian infectious bronchitis virus I-400, inactivated avian infectious bronchitis virus M41.

9. A vaccine as claimed in any of claims 1-7 which contains in addition to said inactivated avian infectious bronchitis virus I-400, inactivated avian infectious bronchitis virus M41 and inactivated Newcastle disease virus.

10. A vaccine as claimed in any of claims 1-7 which contains in addition to said inactivated avian infectious bronchitis virus I-400, inactivated avian infectious bronchitis virus M41, inactivated Newcastle disease virus and inactivated infectious bursal disease virus.

11. A kit comprising separately multiple dosage units of each of an inactivated vaccine containing inactivated avian infectious bronchitis virus strain I-400 and/or a virus strain which cross-reacts significantly with said strain, and a live vaccine containing a live heterologous avian infectious bronchitis virus.

12. Use of inactivated avian infectious bronchitis virus strain I-400, and/or an inactivated virus strain which

EP 0 186 368 B1

cross-reacts significantly with said strain to prepare a vaccine for use in vaccinating birds against avian infectious bronchitis in combination with an initial vaccination with a live vaccine containing a live heterologous avian infectious bronchitis virus.

Claims for the following Contracting State: AT

1. A process for the preparation of a vaccine containing inactivated avian infectious bronchitis virus strain deposited with the Institut Pasteur under the accession number I-400 and/or an inactivated virus strain which cross-reacts significantly with said Strain the immune response to which in birds is enhanced by priming with a live heterologous strain of avian infectious bronchitis virus, which process comprises incorporating said inactivated virus into an adjuvant or carrier.

2. A process as claimed in claim 1 comprising growing avian infectious bronchitis virus strain I-400 and/or an inactivated virus strain which cross-reacts significantly with said Strain the immune response to which in birds is enhanced by priming with a live heterologous strain of avian infectious bronchitis virus, reacting said virus with an inactivating agent and incorporating the inactivated virus into an adjuvant or carrier.

3. A process as claimed in claim 1 or claim 2 wherein the inactivated avian infectious bronchitis virus is I-400.

4. A process as claimed in any one of claims 1 to 3 wherein the inactivated antigen in an aqueous medium is mixed with aluminium hydroxide or an oil emulsion comprising an oil, an oil-soluble surfactant and a water-soluble surfactant.

5. A process as claimed in claim 4 wherein the adjuvant is an oil emulsion in which the oil is a paraffin oil or squalane.

6. A process as claimed in claim 4 or claim 5 wherein the adjuvant is an oil emulsion in which the ratio of oil to aqueous phase is about 4:1.

7. A process as claimed in any one of claims 1 to 6 comprising incorporating into said adjuvant or carrier inactivated virus obtained from 6 to 8 $\log_{10}$ CD$_{50}$ of the variant strain I-400 and/or said cross-reacting strain per dose of vaccine.

8. A process as claimed in any one of claims 1 to 7 in which, in addition to inactivated avian infectious bronchitis virus strain I-400 and/or an inactivated virus strain which cross-reacts significantly with said Strain, one or more additional inactivated virus strains is incorporated into said adjuvant or carrier.

9. A process as claimed in claim 8 in which the additional inactivated virus strain is inactivated avian infectious bronchitis virus M41.

10. A process as claimed in claim 8 in which the additional inactivated virus strains are inactivated avian infectious bronchitis virus M41 and inactivated Newcastle disease virus.

11. A process as claimed in claim 8 in which the additional inactivated virus strains are inactivated avian infectious bronchitis virus M41, inactivated Newcastle disease virus and inactivated infectious bursal disease virus.

12. Use of inactivated avian infectious bronchitis virus strain I-400, and/or an inactivated virus strain which cross-reacts significantly with said Strain to prepare a vaccine for use in vaccinating birds against avian infectious bronchitis in combination with an initial vaccination with a live vaccine containing a live heterologous avian infectious bronchitis virus.

**Revendications**

1. Vaccins qui contiennent la souche de virus de la bronchite infectieuse aviaire, inactivé, déposée à l'

9

institut Pasteur sous le n° d'accès I-400 et/ou une souche de virus inactivé qui réalise une réaction croisée notable avec ladite souche, à laquelle la réponse immunitaire est augmentée chez des oiseaux par stimulation avec une souche hétérologue vivante du virus de la bronchite infectieuse aviaire.

2. Vaccin suivant la revendication 1, caractérisé en ce que le virus de la bronchite infectieuse aviaire inactivé est I-400.

3. Vaccin suivant la revendication 1 ou la revendication 2, caractérisé en ce qu'on mélange l'antigène inactivé dans un milieu aqueux à de l'hydroxyde d'aluminium ou une oléo-émulsion comprenant une huile, un surfactif ou tensio-actif oléosoluble et un surfactif ou tensio-actif hydrosoluble.

4. Vaccin suivant la revendication 3, caractérisé en ce qu'il comprend, à titre d'adjuvant, une oléo-émulsion dans laquelle l'huile est le squalane ou une huile de paraffine.

5. Vaccin suivant la revendication 3 ou la revendication 4, caractérise en ce qu'il comprend, à titre d'adjuvant, une oléo-émulsion dans laquelle le rapport de l'huile à la phase aqueuse est d'environ 4:1.

6. Vaccin suivant l'une quelconque des revendications 1 à 5, caractérisé en ce qu'il contient le virus inactivé obtenu à partir de 6 à 8 $\log_{10}$ $CD_{50}$ de la souche variante I-400 et/ou de ladite souche à réaction croisée, par dose de vaccin.

7. Vaccin suivant l'une quelconque des revendications 1 à 6, caractérisé en ce qu'il contient, outre la souche de virus de la bronchite infectieuse aviaire inactivé I-400 et/ou une souche de virus inactivé qui réalise une réaction croisée notable avec ladite souche, une ou plusieurs souches de virus inactivés supplémentaires.

8. Vaccin suivant l'une quelconque des revendications 1 à 7, caractérisé en ce qu'il contient, outre ledit virus de la bronchite infectieuse aviaire inactivé I-400, le virus de la bronchite infectieuse aviaire inactivé M41.

9. Vaccin suivant l'une quelconque des revendications 1 à 7, caractérisé en ce qu'il contient, outre ledit virus de la bronchite infectieuse aviaire inactivé I-400, le virus de la bronchite infectieuse aviaire inactivé M41 et le virus de la maladie de Newcastle inactivé.

10. Vaccin suivant l'une quelconque des revendications 1 à 7, caractérisé en ce qu'il contient, outre ledit virus de la bronchite infectieuse aviaire inactivé I-400, le virus de la bronchite infectieuse aviaire inactivé M41, le virus de la maladie de Newcastle inactivé et le virus de la maladie bursale infectieuse inactivé.

11. Trousse comprenant séparément les unités de dosage multiples d'un vaccin inactivé contenant une souche de virus de la bronchite infectieuse aviaire inactivé I-400 et/ou une souche de virus qui réalise une réaction croisée notable avec ladite souche et un vaccin vivant contenant un virus de la bronchite infectieuse aviaire hétérologue vivant.

12. Utilisation d'une souche de virus de la bronchite infectieuse aviaire inactivé I-400 et/ou d'une souche de virus inactivé qui réalise une réaction croisée notable avec ladite souche, en vue de préparer un vaccin destiné à la vaccination d'oiseaux contre la bronchite infectieuse aviaire, en combinaison avec une vaccination initiale par un vaccin vivant contenant un virus de la bronchite infectieuse aviaire hétérologue vivant.

Revendications pour les Etats contractants suivants : AT

1. Procédé de préparation d'un vaccin qui contient la souche de virus de la bronchite infectieuse aviaire, inactivé, déposée à l'institut Pasteur sous le n° d'accès I-400 et/ou une souche de virus inactivé qui réalise une réaction croisée notable avec ladite souche, à laquelle la réponse immunitaire est augmentée chez des oiseaux par stimulation avec une souche hétérologue vivante du virus de la bronchite infectieuse aviaire, caractérisé en ce que l'on incorpore ledit virus inactivé dans un adjuvant ou un véhicule ou excipient.

2. Procédé suivant la revendication 1, caractérisé en ce que l'on fait croître une souche de virus de la bronchite infectieuse aviaire I-400 et/ou une souche de virus inactivé qui réalise une réaction croisée notable avec ladite souche, à laquelle la réponse immunitaire est augmentée chez les oiseaux par stimulation avec une souche hétérologue vivante de virus de la bronchite infectieuse aviaire, on fait réagir le virus en question sur un agent inactivant et on incorpore le virus inactivé à un adjuvant ou véhicule ou excipient.

3. Procédé suivant la revendication 1 ou la revendication 2, caractérisé en ce que le virus de la bronchite infectieuse aviaire inactivé est I-400.

4. Procédé suivant l'une quelconque des revendications 1 à 3, caractérisé en ce qu'on mélange l'antigène inactivé dans un milieu aqueux à de l'hydroxyde d'aluminium ou une oléo-émulsion comprenant une huile, un surfactif ou tensio-actif oléosoluble et un surfactif ou tensioactif hydrosoluble.

5. Procédé suivant la revendication 4, caractérisé en ce que l'adjuvant est une oléo-émulsion dans laquelle l'huile est le squalane ou une huile de paraffine.

6. Procédé suivant la revendication 4 ou la revendication 5, caractérisé en ce que l'adjuvant est une oléo-émulsion dans laquelle le rapport de l'huile à la phase aqueuse est d'environ 4:1.

7. Procédé suivant l'une quelconque des revendications 1 à 6, caractérisé en ce que l'on incorpore audit adjuvant ou véhicule ou excipient, un virus inactivé obtenu à partir de 6 à 8 $\log_{10}$ $CD_{50}$ de la souche variante I-400 et/ou de ladite souche à réaction croisée, par dose de vaccin.

8. Procédé suivant l'une quelconque des revendications 1 à 7, caractérisé en ce qu'outre la souche de virus de la bronchite infectieuse aviaire inactivé I-400 et/ou une souche de virus inactivé qui réalise une réaction croisée notable avec ladite souche, on incorpore une ou plusieurs souches de virus inactivé supplémentaires dans ledit adjuvant ou véhicule ou excipient.

9. Procédé suivant la revendication 1, caractérisé en ce que la souche de virus inactivé supplémentaire est le virus de la bronchite infectieuse aviaire inactivé M41.

10. Procédé suivant la revendication 1, caractérisé en ce que les souches de virus inactivé supplémentaires sont le virus de la bronchite infectieuse aviaire inactivé M41 et le virus de la maladie de Newcastle inactivé.

11. Procédé suivant la revendication 8, caractérisé en ce que les souches de virus inactivé supplémentaires sont le virus de la bronchite infectieuse aviaire inactivé M41, le virus de la maladie de Newcastle inactivé et le virus de la maladie bursale infectieuse inactivé.

12. Utilisation d'une souche de virus de la bronchite infectieuse aviaire inactivé et/ou d'une souche de virus inactivé qui réalise une réaction croisée notable avec ladite souche, en vue de préparer un vaccin destiné à la vaccination d'oiseaux contre la bronchite infectieuse aviaire, en combinaison avec une vaccination initiale par un vaccin vivant contenant un virus de la bronchite infectieuse aviaire hétérologue vivant.

**Ansprüche**

1. Vaccine, enthaltend inaktiviertes infektiöse-Vogelbronchitis-Virus, Stamm hinterlegt beim Institut Pasteur unter der Hinterlegungsnummer I-400, und/oder einen inaktivierten Virusstamm, der mit diesem Stamm signifikant kreuzreagiert, wobei die Immunreaktion zu ihm in Vögeln durch Vorbereitung mit einem lebenden heterologen Stamm von infektiöse-Vogelbronchitis-Virus erhöht wird.

2. Vaccine gemäß Anspruch 1, worin das inaktivierte infektiöse-Vogelbronchitis-Virus I-400 ist.

3. Vaccine gemäß Anspruch 1 oder Anspruch 2, worin das inaktivierte Antigen in einem wäßrigen Medium gemischt ist mit Aluminiumhydroxid oder einer Ölemulsion enthaltend ein Öl, ein öllösliches, oberflä-

11

chenaktives Mittel und ein wasserlösliches, oberflächenaktives Mittel.

4. Vaccine gemäß Anspruch 3, welche als Hilfsmittel eine Ölemulsion enthält, worin das Öl ein Paraffinöl oder Squalan ist.

5. Vaccine gemäß Anspruch 3 oder Anspruch 4, welche als Hilfsmittel eine Ölemulsion enthält, worin das Verhältnis von Öl zu wäßriger Phase etwa 4:1 beträgt.

6. Vaccine gemäß einem der Ansprüche 1 bis 5, welche inaktiviertes Virus enthält, erhalten aus 6 bis 8 $\log_{10}$ $CD_{50}$ des Variantenstamms I-400 und/oder diesen kreuzreagierenden Stamm pro Dosis der Vaccine.

7. Vaccine gemäß einem der Ansprüche 1 bis 6, welche zusätzlich zu dem inaktivierten infektiöse Vogelbronchitis-Virus Stamm I-400 und/oder einem inaktivierten Virusstamm, der mit diesem Stamm signifikant kreuzreagiert, einen oder mehrere zusätzliche inaktivierte Virusstämme enthält.

8. Vaccine gemäß einem der Ansprüche 1 bis 7, welche zusätzlich zu dem inaktivierten infektiöse-Vogelbronchitis-Virus I-400 inaktiviertes infektiöse Vogelbronchitis-Virus M41 enthält.

9. Vaccine gemäß einem der Ansprüche 1 bis 7, welche zusätzlich zu dem genannten inaktivierten infektiöse-Vogelbronchitis-Virus I-400 inaktiviertes infektiöse-Vogelbronchitis-Virus M41 und inaktiviertes Newcastle-Kranheits-Virus enthält.

10. Vaccine gemäß einem der Ansprüche 1 bis 7, welche zusätzlich zu dem genannten inaktivierten infektiöse-Vogelbronchitis-Virus I-400 inaktiviertes infektiöse Vogelbronchitis-Virus M41, inaktiviertes Newcastle-Krankheits-Virus und inaktiviertes infektiöse-Schleimbeutelerkrankung-Virus enthält.

11. Kit, umfassend getrennt mehrere Dosierungseinheiten von jeweils einer inaktivierten Vaccine, enthaltend inaktiviertes infektiöse-Vogelbronchitis-Virus Stamm I-400 und/oder einen Virusstamm, der mit diesem Stamm signifikant kreuzreagiert, und eine Lebend-Vaccine, enthaltend ein lebendes heterologes infektiöse-Vogelbronchitis-Virus.

12. Verwendung des inaktivierten infektiöse Vogelbronchitis-Virus Stamm I-400 und/oder eines inaktivierten Virusstammes, der mit diesem Stamm signifikant kreuzreagiert, zur Herstellung einer Vaccine zur Verwendung beim Impfen von Vögeln gegen infektiöse Vogelbronchitis in Kombination mit einer Erstimpfung mit einer Lebendvaccine, enthaltend ein lebendes heterologes infektiöse Vogelbronchitis-Virus.

Patentansprüche für folgende Vertragsstaaten: AT

1. Verfahren zur Herstellung einer Vaccine, enthaltend inaktiviertes infektiöse-Vogelbronchitis-Virus, Stamm hinterlegt beim Institut Pasteur unter der Hinterlegungsnummer I-400, und/oder einen inaktivierten Virusstamm, der mit diesem Stamm signifikant kreuzreagiert, wobei die Immunreaktion zu ihm in Vögeln durch Vorbereitung mit einem lebenden heterologen Stamm von infektiöse-Vogelbronchitis-Virus erhöht wird, wobei das Verfahren die Einverleibung dieses inaktivierten Virus in ein Hilfsmittel oder Träger umfaßt.

2. Verfahren gemäß Anspruch 1, dadurch gekennzeichnet, daß infektiöse-Vogelbronchitis-Virus Stamm I-400 und/oder ein inaktivierter Virusstamm, der mit diesem Stamm signifikant kreuzreagiert gezüchtet wird, wobei die Immunreaktion zu ihm in Vögeln durch Vorbereitung mit einem lebenden heterologen Stamm von infektiöse-Vogelbronchitis-Virus erhöht wird, Reaktion dieses Virus mit einem inaktivierenden Mittel und Einverleibung des inaktivierten Virus in ein Hilfsmittel oder Träger.

3. Verfahren gemäß Anspruch 1 oder Anspruch 2, worin das inaktivierte infektiöse-Vogelbronchitis-Virus I-400 ist.

4. Verfahren gemäß einem der Ansprüche 1 bis 3, worin das inaktivierte Antigen in einem wäßrigen Medium mit Aluminiumhydroxid oder einer Ölemulsion, umfassend ein Öl, ein öllösliches oberflächenaktives Mittel und ein wasserlösliches oberflächenaktives Mittel, gemischt wird.

5. Verfahren gemäß Anspruch 4, worin das Hilfsmittel eine Ölemulsion ist, in der das Öl ein Paraffinöl oder Squalan ist.

6. Verfahren gemäß Anspruch 4 oder Anspruch 5, worin das Hilfsmittel eine Ölemulsion ist, in der das Verhältnis von Öl zu wäßriger Phase etwa 4:1 beträgt.

7. Verfahren gemäß einem der Ansprüche 1 bis 6, dadurch gekennzeichnet, daß diesem Hilfsmittel oder Träger inaktiviertes Virus einverleibt wird, erhalten aus 6 bis 8 $\log_{10}$ $CD_{50}$ des Variantenstamms I-400 und/oder dieses kreuzreagierden Stamms pro Dosis der Vaccine.

8. Verfahren gemäß einem der Ansprüche 1 bis 7, worin zusätzlich zu dem inaktivierten infektiöse-Vogelbronchitis-Virusstamm I-400 und/oder einem inaktivierten Virusstamm, der mit diesem Stamm signifikant kreuzreagiert, ein oder mehrere zusätzliche inaktivierte Virusstämme in dieses Hilfsmittel oder Träger einverleibt werden.

9. Verfahren gemäß Anspruch 8, worin der zusätzliche inaktivierte Virusstamm inaktiviertes infektiöse-Vogelbronchitis-Virus M41 ist.

10. Verfahren gemäß Anspruch 8, worin die zusätzlichen inaktivierten Virusstämme inaktiviertes infektiöse-Vogelbronchitis-Virus M41 und inaktiviertes Newcastle-Krankheits-Virus ist.

11. Verfahren gemäß Anspruch 8, worin die zusätzlichen inaktivierten Virusstämme inaktiviertes infektiöse-Vogelbronchitis-Virus M41, inaktiviertes Newcastle-Krankheits-Virus und inaktiviertes infektiöses-Schleimbeutel-Krankeits-Virus ist.

12. Verwendung von inaktiviertem infektiöse-Vogelbronchitis-Virus Stamm I-400 und/oder eines inaktivierten Virusstamms, der mit diesem Stamm signifikant kreuzreagiert, zur Herstellung einer Vaccine zur Verwendung zur Impfung von Vögeln gegen infektiöse Vogelbronchitis mit einer Erstimpfung mit einer Lebendvaccine, enthaltend ein lebendes, heterologes infektiöses-Vogelbronchitis-Virus.